(19) Europäisches Patentamt
European Patent Office
Office européen des brevets

(11) EP 1 352 935 A1

(12) EUROPÄISCHE PATENTANMELDUNG

(43) Veröffentlichungstag:
**15.10.2003 Patentblatt 2003/42**

(51) Int Cl.[7]: **C09D 183/04**, C09D 183/14, C08G 77/04

(21) Anmeldenummer: **03006883.7**

(22) Anmeldetag: **31.03.2003**

(84) Benannte Vertragsstaaten:
**AT BE BG CH CY CZ DE DK EE ES FI FR GB GR HU IE IT LI LU MC NL PT RO SE SI SK TR**
Benannte Erstreckungsstaaten:
**AL LT LV MK**

(30) Priorität: **11.04.2002 DE 10215941**

(71) Anmelder: **Bayer Aktiengesellschaft
51368 Leverkusen (DE)**

(72) Erfinder: **Mager, Michael, Dr.
51375 Leverkusen (DE)**

(54) **Anorganische UV-Absorber enthaltende Bindemittel**

(57) Die vorliegende Erfindung betrifft anorganische UV-Absorber enthaltende Bindemittel sowie daraus hergestellte Beschichtungen zum dauerhaften Schutz von Materialien, insbesondere Kunststoffen, vor photochemischer Degradation.

EP 1 352 935 A1

## Beschreibung

Die vorliegende Erfindung betrifft anorganische UV-Absorber enthaltende Bindemittel sowie daraus hergestellte Beschichtungen zum dauerhaften Schutz von Materialien, insbesondere Kunststoffen, vor photochemischer Degradation.

Viele Materialien wie Kunststoffe oder Naturstoffe (Holz) müssen für den Einsatz im Außenbereich mit einer geeigneten Beschichtung gegen photochemische Degradation geschützt werden. In erster Linie bietet sich dazu eine Beschichtung der Oberfläche über einen nasschemischen Prozess an, bei flächigen Substraten wie KunststoffPlatten ist es allerdings auch möglich durch (beidseitige) Coextrusion eines UV-Absorber-haltigen Kunststoffs einen entsprechenden Schutz zu erreichen. Die nasschemische Applikation einer Beschichtung, insbesondere einer anorganischen Beschichtung, weist allerdings den Vorteil auf, dass dadurch in der Regel zusätzlich auch die Chemikalienbeständigkeit und Kratzfestigkeit des Kunststoffes verbessert werden. Die Coextrusion bietet diese Vorteile nicht, ist demgegenüber aber technisch wesentlich einfacher durchzuführen.

In den (nasschemisch) applizierten Beschichtungen sowie auch in den coextrudierten Schichten werden üblicherweise organische UV-Absorber eingesetzt, die das darunter liegenden Material meist zufriedenstellend schützen (siehe z.B. Paint & Coatings Industry 2001, July, 64-76). Bei sehr lange dauernder Belastung durch UV-Strahlung (Sonnenlicht) werden organische UV-Absorber jedoch langsam abgebaut und verlieren somit ihre Schutzwirkung. Durch Witterungseinflüsse (Feuchtigkeit, höhere Temperaturen) kann es darüber hinaus zum Verlust von UV-Absorber durch Migration und Auswaschen kommen.

Anorganische UV-Absorber wie beispielsweise Titandioxid, Cerdioxid oder Zinkoxid weisen hingegen die genannten Nachteile organischer UV-Absorber nicht auf.

Sie werden nicht photochemisch abgebaut, weder Ausgewaschen noch bei thermischer Belastung ausgetragen.

Ist die Partikelgröße der eingesetzten anorganischen UV-Absorber genügend klein, so gelingt es hochtransparente Beschichtungen daraus herzustellen. Prinzipiell können hierfür organische und anorganische Bindemittel eingesetzt werden. Ein Beispiel für die Einarbeitung von nano-Cerdioxid in Polyacrylate, die dann als Haftvermittler auf Thermoplasten eingesetzt werden, ist in EP 0 732 356 A2 gegeben. Aufgrund der hohen Oberfläche von Titandioxid-, Cerdioxid- und Zinkoxid-Nanopartikeln kommt es jedoch häufig zu einer photochemischen Schädigung und schließlich zu einem Abbau der die anorganischen UV-Absorber umgebenden Matrix. Dies führt dann u.a. zu einem Haftungsverlust der Beschichtung zum Substrat (Kunststoff).

Durch die Verwendung anorganischer Bindemittel, insbesondere von Sol-Gel-Materialien, kann dieser Abbau vermieden werden. Die überwiegend anorganische Matrix wird in den meisten Fällen durch die Photoaktivität der genannten Nanopartikeln nicht geschädigt.

Einige UV-Schutzformulierungen auf Basis von Titandioxid-, Cerdioxid- und Zinkoxid-Nanopartikeln in Sol-Gel-Materialien sind mittlerweile Stand der Technik. So sind in US-A 4,799,963 transparente Beschichtungszusammensetzungen beschrieben, die aus teilweise hydrolysierten (Organo-) Alkoxiden der Formel $R_xM(OR)_z$, und kolloidalem Cerdioxid bestehen, wobei in der angegebenen Formel R ein organischer Rest ist, x gleich 0 sein kann, aber kleiner z ist und M für Si, Al, Ti oder Zr bzw. Mischungen daraus steht. Die in US-A 4,799,963 beschriebenen Zusammensetzungen weisen jedoch den Nachteil auf, dass die erfindungsgemäßen Mischungen der Organosilane $CH_3Si(OC_2H_5)_3$ und $(CH_3)_2Si(OC_2H_5)_2$, Beispiel 1, bzw. $CH_3Si(OCH_3)_3$ und $(CH_3)_2Si(OC_2H_5)_2$, Beispiel 2, lediglich Feststoffanteile von ca. 6 Gew.-% nano-$CeO_2$, berechnet auf die vollständig hydrolysierten und kondensierten Methylalkoxysilane, aufweisen. Bei Verwendung von $Si(OC_2H_5)_4$, Beispiel 3, wird zwar ein deutlich höherer Anteil an nano-$CeO_2$ erreicht, jedoch sind die daraus hergestellten Beschichtungen so spröde, dass sie nur auf Glas und nicht auf Kunststoff appliziert werden können. Auch durch Verwendung des funktionellen Organosilans 3-Glycidoxypropyltrimethoxysilan, Beispiel 4, lässt sich der Gehalt von nano-$CeO_2$ im Feststoff auf lediglich ca. 13 Gew.-% steigern. Da die beschriebenen Beschichtungen nur in begrenzten Schichtdicken appliziert werden können (Rissbildung, Abplatzen) ist die Schutzwirkung gegenüber längerwelliger UV-Strahlung (oberhalb von etwa 300 nm) aufgrund des begrenzten Gehalts an nano-$CeO_2$ jedoch so gering, dass ein dauerhafter Schutz des Untergrundes nicht gegeben ist. Sonnenlicht enthält nur UV-Strahlung oberhalb von etwa 300 nm, die Schutzwirkung oberhalb des genannten Werts ist daher wesentlich für alle Außenanwendungen.

In DE-A 198 58 998 sind weitere Beschichtungszusammensetzungen mit nano-$CeO_2$ beschrieben, wobei neben Epoxygruppen-haltigen Silanen zusätzlich teilchenförmiges Böhmit, eine weitere hydrolysierbare Siliciumverbindung sowie eine hydrolysierbare Aluminiumverbindung enthalten sind. Zwar konnten in den gegebenen Beschichtungszusammensetzungen, gemäß den Beispielen, Gehalte an nano-$CeO_2$ von bis zu 25 Gew.-% realisiert werden und die Bewitterung (Suntest) von entsprechend beschichtetem Polycarbonat (Makrolon® 2808) zeigt eine gewisse UV-Schutzwirkung. Die erreichten Gelbwerte (Y.I.) liegen jedoch alle oberhalb von 3,5 und sind damit für viele Anwendungen noch zu hoch.

In EP 0 947 520 A1 sind Mischungen aus polyfunktionellen Organosilanen und aluminium- und/oder borhaltigen Verbindungen beschrieben, die darüber hinaus auch anorganische Partikeln wie z.B. die Oxide der Elemente B, Al, Si, Ti, Zr und Ce enthalten können. Es findet sich jedoch keinerlei Hinweis auf die Eignung solcher Beschichtungen als UV-Schutz. In Kombination mit nano-$CeO_2$, neigen die Beschichtungen auf Grund ihrer großen mechanischen Härte und der damit verbundenen Sprödigkeit zudem stark zu Spannungsrissen bei Bewitterung und sind damit für Außenanwendungen ungeeignet.

Aufgabe der vorliegenden Erfindung war daher die Bereitstellung von UV-Schutzformulierungen, die nach Applikation auf ein geeignetes Substrat, wie z.B. einen Kunststoff, zu transparenten Beschichtungen ausgehärtet werden können, und eine effektive und dauerhafte UV-Schutzwirkung, bei hoher Witterungsbeständigkeit zeigen.

Es wurde nun überraschenderweise gefunden, dass durch die Verwendung polyfunktioneller Organosilane UV-Schutzformulierungen mit mehr als 15 Gew.-% nano-$CeO_2$ herstellbar sind, und diese selbst bei einem Gehalt von 30 Gew.-% und mehr nano-$CeO_2$ zu transparenten Beschichtungen ausgehärtet werden können, welche eine effektive und dauerhafte UV-Schutzwirkung, bei hoher Witterungsbeständigkeit zeigen.

Gegenstand der vorliegenden Erfindung sind daher Zusammensetzungen enthaltend, berechnet auf die Feststoffe bei vollständiger Hydrolyse und Kondensation,

A) 30-85 Gew.-% mindestens eines polyfunktionellen Organosilans sowie
B) 15-70 Gew.-% nano-$CeO_2$,

wobei die Zusammensetzungen weniger als 0,1 Gew. %, bevorzugt 0 %, eines Elements der dritten Hauptgruppe, bzw. Verbindungen dieser Elemente, enthalten.

Ein weiterer Gegenstand der vorliegenden Erfindung ist ein Verfahren zur Herstellung von UV-Schutzformulierungen enthaltend 30-85 Gew.-% mindestens eines polyfunktionellen Organosilans und 15-70 Gew.-% nano-$CeO_2$ sowie deren Verwendung zur Beschichtung von Oberflächen, insbesondere Oberflächen von Kunststoffen.

Geeignete polyfunktionelle Organosilane, die in den erfindungsgemäßen UV-Schutzformulierungen eingesetzt werden können, sind Monomere, Oligomere und/oder Polymere, dadurch gekennzeichnet, dass mindestens 2 Siliciumatome mit hydrolysierbaren und/oder kondensationsvernetzenden Gruppen über jeweils mindestens eine Si, C-Bindung an eine die Siliciumatome verknüpfende Baueinheit gebunden sind. Aufgrund ihrer guten Verträglichkeit mit nano-$CeO_2$ sind polyfunktionelle Organosilane mit mindestens 3, besser noch mit mindestens 4 Siliciumatomen mit hydrolysierbaren und/oder kondensationsvernetzenden Gruppen besonders für die erfindungsgemäßen UV-Schutzformulierungen geeignet. Als hydrolysierbare Gruppen sind insbesondere Alkoxy- oder Aryloxygruppen geeignet, bevorzugt seien Alkyloxygruppen, wie Methyloxy-, Ethyloxy-, Propyloxy- oder Butyloxy, genannt. Kondensationsvernetzende Gruppen sind insbesondere Silanolgruppen (Si-OH). Als verknüpfende Baueinheiten im Sinne der Erfindung, seien sowohl einzelne Atome als auch Moleküle genannt. Molekulare Baueinheiten können z.B. lineare oder verzweigte $C_1$-$C_{20}$-Alkylenketten, $C_5$-$C_{10}$-Cycloalkylenreste oder $C_6$-$C_{12}$-aromatische Reste, wie z.B. Phenyl-, Naphtyl- oder Biphenylreste, sein. Die genannten Reste können ein- oder mehrfach substituiert sein und können insbesondere auch innerhalb der Ketten oder Ringe Heteroatome, wie z.B. Si, N, P, O oder S, enthalten.

Besonders witterungsbeständige Beschichtungen erhält man, wenn die verknüpfende Baueinheit der polyfunktionellen Organosilane aus linearen, verzweigten, cyclischen oder käfigförmigen Carbosilanen, Carbosiloxanen oder Siloxanen besteht. Beispiele für solche polyfunktionellen Organosilane sind in den allgemeinen Formeln (I), (II) und (III) gezeigt.

$$[(R^1O)_{3-a}(R^2)_aSi(CH_2)_e]_c\text{-}X\text{-}[(CH_2)_fSi(OR^3)_{3-b}(R^4)_b]_d \qquad (I)$$

in welcher

| | |
|---|---|
| $R^1$, $R^2$, $R^3$ und $R^4$ | unabhängig voneinander für $C_1$-$C_8$-Alkylreste oder Phenylreste, bevorzugt Methyl-, Ethyl- oder Phenylreste stehen, |
| a, b | unabhängig voneinander 0, 1 oder 2, bevorzugt 0 oder 1 bedeuten, sowie |
| c, d bzw. e, f | unabhängig voneinander größer oder gleich 1, bevorzugt größer oder gleich 2 sind, und |
| X | als verbrückende Baueinheit für ein lineares, verzweigtes, cyclisches oder käfigförmiges Siloxan, Carbosilan oder Carbosiloxan, bevorzugt ein cyclisches oder käfigförmiges Siloxan, Carbosilan oder Carbosiloxan steht. |

Besonders bevorzugt werden cyclische Carbosiloxane der allgemeinen Formel (II) eingesetzt,

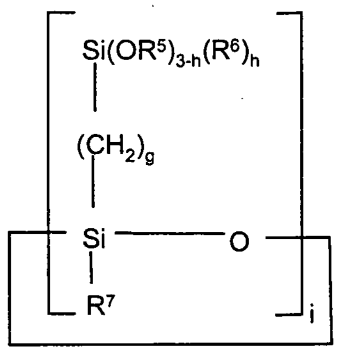

(II)

in welcher

| | |
|---|---|
| $R^5$, $R^6$ und $R^7$ | unabhängig voneinander für $C_1$-$C_4$-Alkylreste stehen, |
| h | für 0, 1 oder 2, bevorzugt 0 oder 1 steht, sowie |
| g | für eine ganze Zahl von 1 bis 4, bevorzugt 2 steht, und |
| i | für eine ganze Zahl von 3 bis 10, bevorzugt 4, 5 oder 6 steht. |

Beispielhaft seien als cyclische Carbosiloxane Verbindungen der Formeln (IIIa) bis (IIIe) genannt, worin $R^8$ für Methyl oder Ethyl steht:

(IIIa) *cyclo*-$\{OSi[(CH_2)_2Si(OH)(CH_3)_2]\}_4$

(IIIb) *cyclo*-$\{OSi[(CH_2)_2Si(OR^8)(CH_3)_2]\}_4$

(IIIc) *cyclo*-$\{OSi[(CH_2)_2Si(OH)_2(CH_3)]\}_4$

(IIId) *cyclo*-$\{OSi[(CH_2)_2Si(OR^8)_2(CH_3)]\}_4$

(IIIe) *cyclo*-$\{OSi[(CH_2)_2Si(OR^8)_3]\}_4$.

Die in der WO 98/52992 (Seite 2)offenbarten Oligomere der genannten cyclischen Carbosiloxane können selbstverständlich ebenso im erfindungsgemäßen Verfahren als polyfunktionelle Organosilane eingesetzt werden. Es ist ebenfalls möglich, Mischungen verschiedener cyclischer monomerer oder auch oligomerer Carbosiloxane einzusetzen.

Zur Herstellung transparenter Beschichtungen ist es notwendig, dass in den erfindungsgemäßen UV-Schutzformulierungen nano-$CeO_2$ eingesetzt wird, welches aus Teilchen mit einem mittleren Teilchen-Durchmesser von kleiner 100 nm, besser von kleiner als 50 nm besteht. Die Bestimmung der Teilchengrößenverteilung kann z.B. mit Ultrazentrifugation erfolgen. Stand der Technik sind wässrige oder organische Dispersionen mit den genannten Teilchengrößen und einer Konzentration von bis zu 50 Gew.-%. Insbesondere wässrige Dispersionen von nano-$CeO_2$, die gegebenenfalls noch geringe Mengen an Dispersionstabilisatoren enthalten können, werden in vorteilhafter Weise zur Herstellung der erfindungsgemäßen Beschichtungszusammensetzung eingesetzt. Typische Beispiele sind Dispersionen von nano-$CeO_2$ mit einer mittleren Teilchengröße von ca. 10-50 nm, einem Gehalt von 10 bis 40 Gew.-% und einem Zusatz eines sauren Dispersionsstabilisators wie Essigsäure in einer Konzentration von ca. 1 bis 10 Gew.-%. Falls notwendig, kann die Konzentration wässriger oder organischer nano-$CeO_2$-Dispersionen durch gängige Verfah-

ren wie Destillation erhöht werden.

Die Gewichtsanteile des in der erfindungsgemäßen UV-Schutzformulierung enthaltenen, polyfunktionellen Organosilans, bzw. der Mischung entsprechender Organosilane, und des nano-Cerdioxids sind berechnet (nicht gemessen) und beziehen sich auf die Feststoffe. Bei der Berechnung des Gewichtsanteils des polyrunktionellen Organosilans, bzw. der Mischung entsprechender Organosilane, wird dabei angenommen, dass eine vollständige Hydrolyse der hydrolysierbaren Gruppen zu Si-OH-Gruppen erfolgt und diese dann vollständig unter Bildung von Si-O-Si-Bindungen kondensieren. Die Berechnung des Feststoffgehalts bei vollständiger Hydrolyse und Kondensation sei in nachfolgender Gleichung am Beispiel des polyfunktionellen Organosilans (III d), mit $R^8$=Ethyl, verdeutlicht.

$$882\text{ g (1 mol) } \textit{cyclo}\text{-}\{OSi[(CH_2)_2Si(OC_2H_5)_2(CH_3)]\}_4 + 72\text{ g (4 mol) } H_2O \rightarrow$$

$$585\text{ g "}\textit{cyclo}\text{-}\{OSi[(CH_2)_2Si(O_{1/2})_2(CH_3)]\}_4\text{" + 369 g (8 mol) Ethanol}$$

Werden gemäß obenstehender Gleichung beispielsweise 882 g des polyfunktionellen Organosilans (III d), mit $R^8$=Ethyl, und 300 g nano-$CeO_2$ zur Herstellung der erfindungsgemäßen Mischung eingesetzt, so errechnet sich der Feststoffgehalt von nano-$CeO_2$ nach vollständiger Hydrolyse und Kondensation des polyfunktionellen Organosilans wie folgt:

$$300\text{ g} / (300\text{ g} + 585\text{ g}) * 100 = 34\text{ Gew.-\% nano-}CeO_2.$$

Für das Hydrolyse- und Kondensationsprodukt des polyfunktionellen Organosilans gilt entsprechend:

$$585\text{ g} /(585\text{ g} + 300\text{ g}) * 100 = 66\text{ Gew.-\% "}\textit{cyclo}\text{-}\{OSi[(CH_2)_2Si(O_{1/2})_2(CH_3)]\}_4$$

Werden zusätzlich weitere Verbindungen wie beispielsweise Siliciumalkoxide eingesetzt, von welchen nach vollständiger Hydrolyse und Kondensation ein berechneter Feststoff von z.B. 20 g verbleiben, so errechnen sich die Anteile entsprechend:

$$300\text{ g} / (300\text{ g} + 585\text{ g} + 20\text{g}) * 100 = 33{,}15\text{ Gew.-\% nano-}CeO_2,$$

$$585\text{ g} / (300\text{ g} + 585\text{ g} + 20\text{g}) * 100 = 64{,}64\text{ Gew.-\% "}\textit{cyclo}\text{-}$$

$$\{OSi[(CH_2)_2Si(O_{1/2})_2(CH_3)]\}_4,$$

und

$$20\text{ g} / (300\text{ g} + 585\text{ g} + 20\text{g}) * 100 = 2{,}21\text{ Gew.-\%}$$

weitere Verbindung.

Analog wird mit evtl. vorhandenen weiteren Komponenten verfahren.

Beispielsweise zur Verbesserung der mechanischen Eigenschaften, können die erfindungsgemäßen UV-Schutzformulierungen zusätzlich Siliciumalkoxide der Formel (IV) enthalten, wobei die Mischungen, berechnet auf die Feststoffe nach vollständiger Hydrolyse und Kondensation, dann folgende Gewichtsanteile enthalten:

a1) 30-60 Gew.-% mindestens eines polyfunktionellen Organosilans
b1) 15-40 Gew.-% nano-$CeO_2$ und
c1) 0-35 Gew.-% mindestens eines Siliciumalkoxids der allgemeinen Formel (IV)

wobei die Zusammensetzungen weniger als 0,1 Gew. %, bevorzugt 0 %, eines Elements der dritten Hauptgruppe, bzw. Verbindungen dieser Elemente, enthalten.

Bevorzugt ist folgende Zusammensetzung, enthaltend:

a2) 40-60 Gew.-% mindestens eines polyfunktionellen Organosilans

b2) 20-40 Gew.-% nano-$CeO_2$ und
c2) 20-35 Gew.-% mindestens eines Siliciumalkoxids der allgemeinen Formel (IV)

wobei die Zusammensetzungen weniger als 0,1 Gew. %, bevorzugt 0 %, eines Elements der dritten Hauptgruppe, bzw. Verbindungen dieser Elemente, enthalten.

Der Anteil an Siliciumalkoxid C) wird analog zum Anteil an polyfunktionellem Organosilan als Anteil am Feststoff nach vollständiger Hydrolyse, also Abspaltung der hydrolysierbaren Alkoxid-Funktionen, und Kondensation theoretisch berechnet.

Die gegebenenfalls in den erfindungsgemäßen Mischungen ebenfalls enthaltenen Siliciumälkoxide entsprechen der nachstehenden Formel (IV),

$$(R^9)_aSi(OR^{10})_{4-a} \qquad \text{(IV)}$$

in welcher

a für 0, 1, 2 oder 3 steht,

$R^9$ für einen gegebenenfalls substituierten Alkyl- oder Arylrest steht, und

$R^{10}$ für einen $C_1$ bis $C_3$-Alkylrest steht.

Besonders bevorzugt sind Siliciumalkoxide der Formel (IV), in welcher

a für 0 oder 1 steht,

$R^9$ für einen Methylrest steht, und

$R^{10}$ für einen Methyl- oder Ethylrest steht.

Beispielhaft seien folgende Siliciumalkoxide der Formeln (Va) bis (Vc) genannt, wobei $R^{11}$ für einen Methyl- oder Ethylrest steht:

$$\text{(Va) } Si(OR^{11})_4$$

$$\text{(Vb) } CH_3\text{-}Si(OR^{11})_3$$

$$\text{(Vc) } C_6H_5\text{-}Si(OR^{11})_3.$$

Zur Herstellung der erfindungsgemäßen UV-Schutzformulierungen wird das polyfunktionelle Organosilan, bzw. die Mischung entsprechender Organosilane, zunächst in einem geeigneten Lösungsmittel gelöst und danach mit Wasser, welches einen sauren oder basischen Katalysator enthält, hydrolysiert. Schließlich wird eine überwiegend wässrige Dispersion von nano-$CeO_2$ zugegeben und der pH-Wert der resultierenden Mischung durch Zugabe von Säure oder Base ggf. noch angepasst. Die gesamten Verfahrensschritte werden bevorzugt unter Rühren durchgeführt.

Zur Herstellung erfindungsgemäßer UV-Schutzformulierungen, die zusätzlich Siliciumalkoxide enthalten, wird in entsprechender Weise verfahren, d.h. das Siliciumalkoxid, bzw. die Mischung entsprechender Siliciumalkoxide, wird zunächst mit dem polyfunktionellen Organosilan, bzw. der Mischung entsprechender Organosilane, in einem geeigneten Lösungsmittel gelöst, danach wird mit Wasser, enthaltend einen sauren oder basischen Katalysator, hydrolysiert, schließlich wird eine überwiegend wässrige Dispersion von nano-$CeO_2$ zugegeben und der pH-Wert der resultierenden Mischung durch Zugabe von Säure oder Base ggf. noch angepasst. Die gesamten Verfahrensschritte werden bevorzugt unter Rühren durchgeführt.

Die Menge an Wasser wird so gewählt, dass mindestens 10 Mol-%, bevorzugt mindestens 25 Mol-%, aller hydrolysierbaren Gruppen des polyfunktionellen Organosilans, bzw. die Mischung entsprechender Organosilane, und ggf. des zusätzlich enthaltenen Siliciumalkoxids bzw. der Mischung entsprechender Siliciumalkoxide, hydrolysiert werden können. Nach der Zugabe der wässrigen nano-$CeO_2$-Dispersion enthält die resultierende UV-Schutzformulierung

bevorzugt soviel Wasser, dass das Mol-Verhältnis Si-OR zu Wasser kleiner 2, bevorzugt kleiner 1 und ganz bevorzugt kleiner 0,5 ist. Si-OR steht für eine hydrolysierbare Gruppe eines polyfunktionellen Organosilans und/oder eines Siliciumalkoxids.

Geeignete Lösungsmittel, die zur Herstellung der erfindungsgemäßen UV-Schutzformulierungen eingesetzt werden können, sind beispielsweise Ketone, Alkohole, Ester und Ether, wobei Alkohole bevorzugt sind. Als Beispiele für geeignete Alkohole seien Methanol, Ethanol, 1-Propanol, 2-Propanol, 1-Butanol, 2-Butanol, 1-Pentanol und 1-Methoxy-2-propanol, 1,2-Ethandiol und n-Butylglycol genannt.

In den erfindungsgemäßen Verfahren wird soviel Lösungsmittel eingesetzt, dass der berechnete Feststoffgehalt (nach vollständiger Hydrolyse und Kondensation) der erfindungsgemäßen UV-Schutzformulierungen zwischen 10 und 60 Gew.-%, bevorzugt zwischen 25 und 50 Gew.-% liegt.

Geeignete saure und basische Katalysatoren, die zur Hydrolyse von polyfunktionellen Organosilanen und Siliciumalkoxiden eingesetzt werden können sind insbesondere Brønsted-Säuren und -Basen, wobei Brønsted Säuren bevorzugt sind. Bevorzugt werden starke Säuren in einer Konzentration von 0,05-1,0 Mol/Liter (Wasser) eingesetzt, wobei sowohl anorganische Säuren wie Salzsäure oder Schwefelsäure, als auch organische Säuren wie p-Toluolsulfonsäure geeignet sind.

Eine ggf. notwendige Anpassung des pH-Werts der nach einem der erfindungsgemäßen Verfahren hergestellten UV-Schutzformulierung, wird durch Zugabe einer geeigneten Menge einer bevorzugt schwachen Säure oder Base erreicht. Der resultierende pH-Wert sollte danach bei 4-8 liegen, was die Lagerstabilität der UV-Schutzformulierungen erheblich verbessert.

Die erfindungsgemäßen UV-Schutzformulierungen können zur Herstellung von Beschichtungen eingesetzt werden, indem man diese nach gängigen Verfahren auf entsprechende Substrate appliziert und danach unter geeigneten Bedingungen aushärtet. Die Applikation kann beispielsweise durch Tauchen, Fluten, Sprühen, Rakeln, Gießen oder Streichen erfolgen; danach wird gegebenenfalls vorhandenes Lösungsmittel verdampft und die Beschichtung bei Raumtemperatur oder erhöhter Temperatur ausgehärtet. Angaben zur Applikation nach gängigen Methoden finden sich beispielsweise in Organic Coatings: Science and Technology, John Wiley & Sons 1994, Kapitel 22, Seiten 65-82.

Die aus den erfindungsgemäßen UV-Schutzformulierungen hergestellten Beschichtungen bieten einen guten Schutz des Substrats vor UV-Strahlung und schützen Oberflächen dauerhaft vor photochemischem Abbau. Sie können daher überall dort eingesetzt werden, wo ein UV-labiles Substrat vor UV-Strahlung, in erster Linie aus dem Sonnenlicht oder aus einer künstlichen Strahlenquelle, geschützt werden soll. Viele Kunststoffe, aber auch Naturstoffe wie Holz, können durch die erfindungsgemäßen Beschichtungen dauerhaft vor photochemischem Abbau geschützt werden. Die Beschichtung von Glas, die ebenfalls möglich ist, dient hingegen nicht dem Schutz des Substrats, sondern der Abschirmung von langwelliger UV-Strahlung (≥300 nm), welche z.B. handelsübliches Fensterglas nahezu vollständig durchdringt.

Aufgrund ihrer hohen Transparenz können die erfindungsgemäßen Beschichtungen insbesondere auch auf transparenten Kunststoffen wie Polycarbonat, Poly(meth)-acrylat, Polyester und Polystyrol sowie deren Copolymere und Mischungen (Blends) eingesetzt werden. In besonders vorteilhafter Weise werden Polycarbonate, vor allem Bisphenol-A-basierte (aromatische) Polycarbonate, vor UV-Strahlung geschützt. Derart dauerhaft vor UV-Strahlung geschütztes Polycarbonat kann dann beispielsweise für die Verglasung von Gebäuden und Fahrzeugen eingesetzt werden, wo über lange Zeiträume eine Vergilbung verhindert werden muss.

Im Falle thermoplastischer Kunststoffe können vor allem extrudierte als auch spritzgegossene Formkörper beschichtet werden, beispielsweise in Form von Folien, Platten sowie überwiegend flächigen Substraten.

Zur Verbesserung der Haftung ist es selbstverständlich möglich, einen geeigneten Haftvermittler einzusetzen, der für eine gute Haftung der erfindungsgemäßen Beschichtungen zum Substrat sorgt. Der Haftvermittler kann der erfindungsgemäßen Mischung zugesetzt werden oder wird als separate Beschichtung auf das Substrat aufgebracht.

Überdies können die aus den erfindungsgemäßen Mischungen erhaltenen Beschichtungen mit weiteren Beschichtungen überlackiert werden, was beispielsweise der Verbesserung der mechanischen Eigenschaften (Kratzfestigkeit) dienen kann.

## Ausführungsbeispiele

Als polyfunktionelles Organosilan wurde in den nachfolgenden Versuchen oligomeres *cyclo*-$\{OSi[(CH_2)_2Si(OC_2H_5)_2(CH_3)]\}_4$, D4-Diethoxid-Oligomer, eingesetzt. Dessen Herstellung erfolgte wie in US-A 6,136,939, Beispiel 2, beschrieben. Nano-$CeO_2$ wurde in Form einer kommerziell erhältlichen (Firma Aldrich), 20 %igen Dispersion, stabilisiert mit 2,5 Gew.-% Essigsäure, eingesetzt. Die verwendete 0,1 N wässrige Lösung von p-Toluolsulfonsäure wurde durch Lösen entsprechender Mengen an p-Toluolsulfonsäure in einer entsprechenden Menge Wasser selbst hergestellt, alle anderen Edukte waren kommerziell erhältlich und wurden ohne weitere Reinigung eingesetzt. Zur Herstellung der Mischungen wurden Glasflaschen entsprechender Größe benutzt, gerührt wurde mit einem Magnetrührstäbchen; die Flaschen wurden soweit möglich verschlossen gehalten.

Als Substrate wurden exdrudierte Polycarbonat-Platten aus Makrolon® 3103 (Bayer AG, Leverkusen) eingesetzt. Vor Beschichtung wurden die Platten auf ein Format von 7,5 x 15 cm zugeschnitten, durch Abspülen mit Isopropanol gereinigt und mit einem Haftvermittler versehen. Der Haftvermittler, ein Alkoxysilan-modifiziertes Polyurethan, wurde wie folgt hergestellt:

a) Herstellung der Polyol-Komponente:
9,24 g Desmophen® 800 und 3,08 g Desmophen® 670 wurden unter Rühren in 3,08 g n-Butylacetat gelöst, danach wurden 0,4 g einer 10 %igen Lösung von Zink(II)octoat in Diacetonalkohol, 0,2 g einer 10 %igen Lösung von Baysilone® OL 17 in Diacetonalkohol sowie 170,5 g Diacetonalkohol zugegeben. Es wurden 186,5 g der klaren, farblosen und lagerstabilen Polyol-Komponente erhalten.
b) Herstellung der Polyisocyanat-Komponente:
462,4 g Desmodur® Z 4470 (70 %ig in n-Butylacetat) wurden mit 27,23 g n-Butylacetat verdünnt, danach wurden innerhalb von ca. 2 h 60,4 g N-$^n$Butylaminopropyltrimethoxysilan so zugetropft, dass die Reaktionstemperatur (Innenthermometer) nicht über 40°C stieg. Nach dem Abkühlen wurden 550 g der klaren, schwach gelben und lagerstabilen Polyisocyanat-Komponente erhalten.
c) Herstellung des verarbeitungsfertigen Haftvermittlers:
Zur Herstellung des verarbeitungsfertigen Haftvermittlers wurden 42,3 g der Komponente a) und 7,7 g der Komponente b) unter Rühren vermischt; die erhaltene klare Lösung wurde innerhalb von einer Stunde verarbeitet.

(Desmophen® 800, Desmophen® 670 und Desmodur® Z 4470 sind Handelsprodukte der Firma Bayer AG, Leverkusen; Baysilone® OL 17 ist ein Verlaufsadditiv der Firma GE Bayer Silicones, Leverkusen; es ist für die Erfindung nicht maßgeblich und kann durch jedes andere, entsprechend wirksame Additiv ersetzt werden).

Der Auftrag des wie beschrieben hergestellten Haftvermittlers erfolgte durch Schleudern (2000 U/min, 20 sec Haltezeit), danach wurde dieser 60 min bei 130°C thermisch behandelt. Die so erzielte Schichtdicke lag typischerweise bei ca. 0,3-0,6 µm. Die Applikation der erfindungsgemäßen UV-Schutzformulierungen erfolgte innerhalb einer Stunde nach Aushärtung des Haftvermittlers.

Die Applikation der erfindungsgemäßen UV-Schutzformulierungen erfolgte durch Schleudern bei verschiedenen Drehzahlen. Angegeben ist die maximale Drehzahl (in U/min); die Haltezeit bei maximaler Drehzahl betrug stets 20 Sekunden.

Die Prüfung der UV-Schutzwirkung der erfindungsgemäßen Beschichtungen erfolgte durch künstliche Bewitterung nach ASTM G 154-97, Cycle 1, unter folgenden Bedingungen: 0,77 W/m$^2$/340 nm, 8 h Bestrahlung bei 60°C Schwarztafeltemperatur, 4 h Kondensation bei 50°C Schwarztafeltemperatur. Der Test wird im folgenden als QUV-A-Test bezeichnet. Beurteilt wurde das Auftreten von Rissen sowie der Gelbwert als Maß der Degradation des Polycarbonats.

**Beispiel 1: Herstellung einer UV-Schutzformulierung mit 20 Gew.-% nano-$CeO_2$**

Unter Rühren wurden 30,5 g D4-Diethoxid-Oligomer und 36,4 g Tetraethylorthosilikat in 54,1 g 1-Methoxy-2-propanol gelöst. Nach 5 min wurde die Mischung dann unter Rühren mit 5,1 g 0,1 N p-Toluolsulfonsäurelösung hydrolysiert, nach 30 min Rühren dann nochmals mit 5,11 g. Nach weiteren 60 min Rühren wurden dann noch 40,3 g nano-$CeO_2$-Dispersion (20 %ig) zugegeben; danach wurde die Mischung noch 60 min gerührt und schließlich noch 48 Stunden bei Raumtemperatur stehengelassen. Es wurde eine klare, gelbe Mischung mit (berechnet) 20,8 Gew.-% nano-$CeO_2$ im Feststoff erhalten.

**Beispiel 2: Herstellung einer Beschichtung mit der Mischung aus Beispiel 1**

Die gemäß Beispiel 1 hergestellte Mischung wurde durch Schleudern (700 U/min) auf mit dem beschriebenen Haftvermittler versehene Polycarbonat-Platten appliziert. Nach 10 min Ablüften bei Raumtemperatur und 60 min Aushärten bei 130°C wurde eine optisch einwandfreie Beschichtung erhalten, die eine Schichtdicke von ca. 3,2-3,5 µm aufwies.

**Beispiel 3: Eignung der Beschichtung aus Beispiel 2 als UV-Schutz für Polycarbonat**

Fünf nach Beispiel 2 beschichtete Polycarbonat-Platten wurden einem QUV-A-Test unterzogen und nach 250, 500, 750 und 1000 h Bewitterungsdauer auf Schäden (Risse, Gelbwert) untersucht. Wie der nachfolgenden Tabelle 1 zu entnehmen ist, zeigten die erfindungsgemäßen Beschichtungen eine hervorragende UV-Schutzwirkung, d. h. es traten keine Risse auf und der Gelbwert b* war kleiner 2.

Tabelle 1

| | Optische Beurteilung (Risse) | | | | Gelbwert b* | | | |
|---|---|---|---|---|---|---|---|---|
| Platten-Nummer | 250 h | 500 h | 750 h | 1000 h | 250 h | 500 h | 750 h | 1000 h |
| 1 | keine | keine | keine | keine | 0,4 | 0,2 | 1,2 | 1,2 |
| 2 | keine | keine | keine | keine | 0,3 | 0,1 | 0,7 | 0,8 |
| 3 | keine | keine | keine | keine | 0,5 | 0,3 | 1,6 | 1,6 |
| 4 | keine | keine | keine | keine | 0,7 | 0,3 | 0,7 | 1,3 |
| 5 | keine | keine | keine | keine | 0,4 | 0,3 | 0,7 | 1,1 |

**Beispiel 4: Herstellung einer UV-Schutzformulierung mit 30 Gew.-% nano-$CeO_2$**

Zunächst wurde die Konzentration der kommerziell erhältlichen nano-$CeO_2$-Dispersion durch Abkondensieren von Wasser am Rotationsverdampfer von 20 Gew.-% auf 30 Gew.-% erhöht.

Nun wurden unter Rühren wurden 30,5 g D4-Diethoxid-Oligomer und 36,4 g Tetraethylorthosilikat in 54,1 g 1-Methoxy-2-propanol gelöst. Nach 5 min wurde die Mischung dann unter Rühren mit 5,1 g 0,1 N p-Toluolsulfonsäurelösung hydrolysiert, nach 30 min Rühren dann nochmals mit 5,11 g. Nach weiteren 60 min Rühren wurden schließlich noch 43,3 g der wie beschrieben konzentrierten nano-$CeO_2$-Dispersion (30 %ig) zugegeben danach wurde die Mischung noch 60 min gerührt und schließlich noch 48 Stunden bei Raumtemperatur stehengelassen. Es wurde eine klare, gelbe Mischung mit (berechnet) 30,0 Gew.-% nano-$CeO_2$ im Feststoff erhalten.

**Beispiel 5: Herstellung einer Beschichtung mit der Mischung aus Beispiel 4**

Die gemäß Beispiel 4 hergestellte Mischung wurde durch Schleudern (700 U/min) auf mit dem beschriebenen Haftvermittler versehene Polycarbonat-Platten appliziert. Nach 10 min Ablüften bei Raumtemperatur und 60 min Aushärten bei 130°C wurde eine optisch einwandfreie Beschichtung erhalten, die eine Schichtdicke von ca. 3,4 µm aufwies.

**Beispiel 6: Eignung der Beschichtung aus Beispiel 5 als UV-Schutz für Polycarbonat**

Fünf nach Beispiel 5 beschichtete Polycarbonat-Platten wurden einem QUV-A-Test unterzogen und nach 250, 500, 750 und 1000 h Bewitterungsdauer auf Schäden (Risse, Gelbwert) untersucht. Wie der nachfolgenden Tabelle 2 zu entnehmen ist, zeigten die erfindungsgemäßen Beschichtungen eine hervorragende UV-Schutzwirkung, d. h. es traten keine Risse auf und der Gelbwert b* war kleiner 2.

Tabelle 2

| | Optische Beurteilung (Risse) | | | | Gelbwert b* | | | |
|---|---|---|---|---|---|---|---|---|
| Platten-Nummer | 250h h | 500 h | 750 h | 1000 h | 250 h | 500 h | 750 h | 1000 h |
| 1 | Keine | keine | keine | keine | 0,2 | 0,2 | 0,5 | 0,5 |
| 2 | Keine | keine | keine | keine | 0,0 | 0,0 | 0,2 | 0,2 |
| 3 | Keine | keine | keine | keine | 0,0 | 0,0 | 0,2 | 0,4 |
| 4 | Keine | keine | keine | keine | 0,1 | 0,2 | 0,4 | 0,4 |
| 5 | Keine | keine | keine | keine | 0,1 | 0,1 | 0,4 | 0,5 |

## Patentansprüche

**1.** Zusammensetzungen enthaltend, berechnet auf die Feststoffe bei vollständiger Hydrolyse und Kondensation,

- 30-85 Gew.-% mindestens eines polyfunktionellen Organosilans sowie
- 15-70 Gew.-% nano-$CeO_2$,

wobei die Zusammensetzungen weniger als 0,1 Gew. % eines Elements der dritten Hauptgruppe, bzw. Verbindungen dieser Elemente, enthalten.

2. Verwendung der Zusammensetzungen gemäß Anspruch 1 als UV-Schutzmittel.

3. Verwendung der Zusammensetzungen gemäß Anspruch 1 zur Beschichtung von Substraten.

4. Verfahren zur Herstellung der Zusammensetzung gemäß Anspruch 1 durch Lösen des polyfunktionellen Organosilans, bzw. der Mischung entsprechender Organosilane, in einem geeigneten Lösungsmittel, anschließende Hydrolyse mit Wasser, welches einen sauren oder basischen Katalysator enthält und Zugabe einer überwiegend wässrigen Dispersion von nano-$CeO_2$.

5. Verfahren zur Beschichtung von Substraten durch Aufbringen einer Schicht aus Zusammensetzung gemäß Anspruch 1, anschließendes Entfernen des Lösungsmittels und abschließendes Aushärten der aufgebrachten Schicht.

6. Verfahren gemäß Anspruch 6, **dadurch gekennzeichnet dass** vor dem Aufbringen der Zusammensetzung gemäß Anspruch 1 ein Haftvermittler aufgebracht wird.

7. Formkörper und Extrudate, **dadurch gekennzeichnet, dass** sie mit einer Beschichtung aus einer Zusammensetzung gemäß Anspruch 1 versehen wurden.

Europäisches Patentamt

# EUROPÄISCHER RECHERCHENBERICHT

Nummer der Anmeldung
EP 03 00 6883

| EINSCHLÄGIGE DOKUMENTE | | | |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (Int.Cl.7) |
| X | EP 0 819 744 A (SHINETSU CHEMICAL CO) 21. Januar 1998 (1998-01-21) * Seite 10, Zeile 16 - Zeile 17; Beispiele * | 1-7 | C09D183/04 C09D183/14 C08G77/04 |
| X | US 6 264 859 B1 (BASIL JOHN D ET AL) 24. Juli 2001 (2001-07-24) * Spalte 1, Zeile 14 - Zeile 15; Anspruch 1; Beispiele * | 1-7 | |
| X | EP 0 545 347 A (PPG INDUSTRIES INC) 9. Juni 1993 (1993-06-09) * Seite 4, Zeile 16 - Zeile 36; Ansprüche; Beispiele * | 1-7 | |
| D,Y | US 4 799 963 A (BASIL JOHN D ET AL) 24. Januar 1989 (1989-01-24) * Beispiele * | 1-7 | |
| Y | DE 198 28 257 A (BAYER AG) 30. Dezember 1999 (1999-12-30) * Seite 4, Zeile 7 - Zeile 29; Ansprüche * | 1-7 | RECHERCHIERTE SACHGEBIETE (Int.Cl.7) C09D C08G C08K C08L |

Der vorliegende Recherchenbericht wurde für alle Patentansprüche erstellt

| Recherchenort | Abschlußdatum der Recherche | Prüfer |
|---|---|---|
| MÜNCHEN | 18. Juni 2003 | Schmitz, V |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)

**ANHANG ZUM EUROPÄISCHEN RECHERCHENBERICHT ÜBER DIE EUROPÄISCHE PATENTANMELDUNG NR.** EP 03 00 6883

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

18-06-2003

| Im Recherchenbericht angeführtes Patentdokument | Datum der Veröffentlichung | Mitglied(er) der Patentfamilie | Datum der Veröffentlichung |
|---|---|---|---|
| EP 0819744 A | 21-01-1998 | JP 10081752 A | 31-03-1998 |
| | | DE 69711284 D1 | 02-05-2002 |
| | | DE 69711284 T2 | 21-11-2002 |
| | | EP 0819744 A2 | 21-01-1998 |
| | | US 6093240 A | 25-07-2000 |
| US 6264859 B1 | 24-07-2001 | US 4799963 A | 24-01-1989 |
| | | US 6355189 B1 | 12-03-2002 |
| | | AU 596702 B2 | 10-05-1990 |
| | | AU 7932087 A | 21-04-1988 |
| | | BR 8705242 A | 24-05-1988 |
| | | CN 87107834 A | 27-07-1988 |
| | | DE 3786864 D1 | 09-09-1993 |
| | | DE 3786864 T2 | 24-02-1994 |
| | | EP 0263428 A2 | 13-04-1988 |
| | | ES 2059338 T3 | 16-11-1994 |
| | | JP 6055925 B | 27-07-1994 |
| | | JP 63123838 A | 27-05-1988 |
| | | KR 9204191 B1 | 30-05-1992 |
| | | YU 182387 A1 | 28-02-1989 |
| EP 0545347 A | 09-06-1993 | US 5316854 A | 31-05-1994 |
| | | DE 69201634 D1 | 13-04-1995 |
| | | DE 69201634 T2 | 19-10-1995 |
| | | EP 0545347 A1 | 09-06-1993 |
| | | ES 2072684 T3 | 16-07-1995 |
| | | HU 3786 A1 | 29-07-1996 |
| | | JP 2086252 C | 23-08-1996 |
| | | JP 6064938 A | 08-03-1994 |
| | | JP 8000720 B | 10-01-1996 |
| US 4799963 A | 24-01-1989 | AU 596702 B2 | 10-05-1990 |
| | | AU 7932087 A | 21-04-1988 |
| | | BR 8705242 A | 24-05-1988 |
| | | CN 87107834 A | 27-07-1988 |
| | | DE 3786864 D1 | 09-09-1993 |
| | | DE 3786864 T2 | 24-02-1994 |
| | | EP 0263428 A2 | 13-04-1988 |
| | | ES 2059338 T3 | 16-11-1994 |
| | | JP 6055925 B | 27-07-1994 |
| | | JP 63123838 A | 27-05-1988 |
| | | KR 9204191 B1 | 30-05-1992 |
| | | US 6264859 B1 | 24-07-2001 |
| | | US 6355189 B1 | 12-03-2002 |
| | | YU 182387 A1 | 28-02-1989 |

EPO FORM P0461

Für nähere Einzelheiten zu diesem Anhang : siehe Amtsblatt des Europäischen Patentamts, Nr.12/82

**ANHANG ZUM EUROPÄISCHEN RECHERCHENBERICHT ÜBER DIE EUROPÄISCHE PATENTANMELDUNG NR.** EP 03 00 6883

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

18-06-2003

| Im Recherchenbericht angeführtes Patentdokument | Datum der Veröffentlichung | Mitglied(er) der Patentfamilie | Datum der Veröffentlichung |
|---|---|---|---|
| DE 19828257 A | 30-12-1999 | DE 19828257 A1 | 30-12-1999 |
| | | AU 4266199 A | 10-01-2000 |
| | | DE 19981131 D2 | 26-04-2001 |
| | | WO 9967185 A2 | 29-12-1999 |

EPO FORM P0461

Für nähere Einzelheiten zu diesem Anhang : siehe Amtsblatt des Europäischen Patentamts, Nr.12/82